# EUROPEAN PATENT APPLICATION

(11) **EP 3 150 193 A1**
(43) Date of publication of application: **05.04.2017**
(21) Application number: 16191477.5
(22) Date of filing: 29.09.2016
(51) Int. Cl.: A61K 8/92, A61Q 19/00, A61P 17/00, A61K 9/00, A61K 8/85, A61K 36/61, A61K 36/54, A61K 35/12

(54) **TOPICAL FORMULATIONS OF LANOLIN FOR THE TREATMENT OF DIAPER RASH**

(30) Priority: 30.09.2015 TR 201512005; 22.10.2015 TR 201513198
(71) Applicant: MONTERO GIDA SANAYI VE TICARET A.S., Istinye, Istanbul 34460 (TR)
(72) Inventor: TÜRKYILMAZ, Ali, 34460 Istanbul (TR); YELKEN, Gülay, 34460 Istanbul (TR); GÜNER, Dicle, 34460 Istanbul (TR); AKAY, Resat, 34460 Istanbul (TR)
(74) Representative: Sevinç, Erkan

(57) **Abstract**

The present invention relates to a topical composition of lanolin comprising polyglyceryl-3- polyricinoleate and one or more excipient.

## Description

### Field of Invention

The present invention relates to a topical composition of lanolin comprising polyglyceryl-3- polyricinoleate and one or more excipient.

### The background of the invention

Diaper rash is a common form of irritation and inflammation of those parts of an infant's or adult's body normally covered by a diaper. This condition is also referred to as diaper dermatitis, napkin dermatitis, napkin rash and nappy rash. It is caused by incontinence and diaper use. It frequently occurs in areas immediately adjacent to the diapered area. Hydrated skin is more prone to mechanical damage and chafing of the skin since an increased coefficient of friction is observed, and it may allow irritants to penetrate the stratum corneum more easily. Not only occlusion but also a higher pH can be an underlying factor, which induces several enzymes-mediated irritations. Alkalinization of the skin increases skin penetration of microorganism and activates fecal enzymes.

Lanolin is a refined derivative of the unctuous fat-like sebaceous secretion of sheep. It consists of a highly complex mixture of esters of high molecular weight aliphatic, steroid or triterpenoid alcohols and fatty acids. The best known uses of lanolin and lanolin derivatives are in medicine, cosmetics and toiletries, which take advantage of these natural protective qualities. Lanolin topical is used to treat or prevent dry skin, itching or other skin irritation caused by conditions such as diaper rash, radiation therapy skin burns, and others. Lanolin penetrates the skin, where it both hydrates and prevents moisture loss. By correcting the skin's moisture balance, lanolin creams help to create smoother, softer skin due to its occlusive properties.

Besides the advanteges of topical lanolin compositions, it has also disadvantages. It is difficult to produce a topical formulation comprising a sufficient dissolved concentration of lanolin to exert its full effect and also to optimize the penetration of the compound into the skin. It is hard to provide a particularly rapid cure because of poor penetration of lanolin into the skin. Moreover, due to stability problem, colour change and undesired smell is occurred.

In addition to ease of release, it is also important that any composition of a pharmaceutically active agent should be stable for long periods of time. Physical, chemical, and microbiological factors play role in the stability of topical compositions. Generally for topical compositions, stability requirements include phase separation, color changes, odor, viscosity (dispensability), preservation, chemical changes (pH, hydrolysis, etc.) and light stability. Other concerns include packaging, weight change, and robustness during manufacture and filling.

In this present invention, polyglyceryl-3- polyricinoleate (Akoline PGPR) and one or more excipient is used in order to increase stability of topical composition of lanolin. Akoline PGPR is a viscous, liquid polyglycerol ester of polycondensed fatty acids from castor oil. It is a very effective non-ionic, water-in-oil natural based emulsifier and dispersion stabiliser, widely used in caring creams and natural cosmetics. It is used in baby-care, natural cosmetics and in dermatological preparations due to its mildness and its contribution to the moisturising properties of the skin care creams.

In addition to further increase the stability of the topical composition, melaleuca alternifolia leaf oil is also used in this present invention. Melaleuca alternifolia leaf oil (tea tree oil) is anti-bacterial and antimicrobial - with powerful healing and anti-itch properties. It is a natural antiseptic and it is also very effective against fungi. Due to its natural and antibacterial properties, it is more preferable than paraben as a preservative in baby care products.

In prior art, there are many patent applications for topical formulations comprising lanolin. They are also widely used in the market. However, there is no topical composition comprising both lanolin and polyglyceryl-3- polyricinoleate (Akoline PGPR). In this present invention, a topical composition of lanolin wherein the composition comprises polyglyceryl-3- polyricinoleate is disclosed to overcome stability problems. In addition to further increase the stability of the composition, melaleuca alternifolia leaf oil is also used in topical composition.

### Detailed description of the invention

The present invention provides a topical composition of lanolin comprising polyglyceryl-3- polyricinoleate and one or more excipient.

In an embodiment, lanolin is used in an amount of between 1.0 - 80.0%, preferably 1.0 - 50.0% and more preferably 10.0 - 25.0% by the weight of total formulation.

The main embodiment of this present invention is to obtain a stable topical composition of lanolin. In this invention, polyglyceryl-3- polyricinoleate (Akoline PGPR) is used in order to increase the stability of topical composition of lanolin. For an effective treatment, topical composition of lanolin should meet some criterias such as optimum pH and viscosity and phase stability. The phase stability of a topical composition characterized by the absence of coalescence of the internal phase, absence of creaming and maintenance of elegance with respect to appearance. A topical composition should be homogenous and active agent should be dispersed in composition homogenously. Unstable topical compositions results as a lack of uniformity of drug distribution and poses skin penetration problems. In this present invention to overcome stability problems of lanolin, polyglyceryl-3- polyricinoleate has been used. It has been found that polyglyceryl-3- polyricinoleate decreases phase separation of topical composition. By using polyglyceryl-3- polyricinoleate, a stable topical composition of lanolin has been developed.

According to this embodiment, in order to achieve stability of the topical composition comprising lanolin, polyglyceryl-3- polyricinoleate is used in a specific amount. Polyglyceryl-3- polyricinoleate is present in an amount of between 0.01 - 20.0%, preferably 0.01 - 15.0% and more preferably 0.1 - 10.0% by the weight of total formulation.

In this embodiment, the ratio of lanolin to polyglyceryl-3- polyricinoleate which is between 1 - 25 (w/w), preferably 1 - 15 (w/w) and more preferably 1 - 5 (w/w), provides phase stability of topical composition.

In an embodiment of this present invention, said topical composition of lanolin and polyglyceryl-3- polyricinoleate is in the form of diaper rash, cream foam, gel, paste, lotion, emulsion, balm or combinations thereof, preferably it is in the form of diaper rash.

According to an embodiment, one or more excipient is selected from the group consisting of preservatives, natural emollients, natural humectants, natural antioxidants, natural emulsifiers and natural surfactants.

Suitable preservatives are selected from the group consisting of melaleuca alternifolia leaf oil, rosmarinus officinalis leaf extract, propylene glycol, phenoxyethanol, methyl paraben, propyl paraben and their salts (such as sodium, potassium), sodium benzoate, citric acid, benzoic acid, butylated hydroxytoluene, boric acid, sorbic acid, benzyl alcohol, benzalconium chloride, parahydroxybenzoic acids, butylated hydroxyanisole, hamamelis virginiana extract, tea tree essential oil, thyme essential oil, grapefruit seed extract, bitter orange extract propolis extract, caprylhydroxamid acid, caprylyl glycol, glycerine and mixtures thereof.

Lanolin can be easily oxidized and providing a suitable medium for the reproduction of microorganisms negatively influence the physical properties and physical stability of that composition. Melaleuca alternifolia leaf oil (tea tree oil) is anti-bacterial and antimicrobial - with powerful healing and anti-itch properties. It is a natural antiseptic and it is also very effective against fungi. Due to its natural and antibacterial properties, it is more preferable than paraben as a preservative in baby care products.

In this present invention, melaleuca alternifolia leaf oil (tea tree oil) is used in order to further increase the stability. Melaleuca alternifolia leaf oil provides physical stability of the topical composition comprising lanolin, as well as being a preservative.

Melaleuca alternifolia leaf oil is present in an amount of between 0.01 - 12.0%, preferably 0.01 - 7.0% and more preferably 0.01 - 5.0% by the weight of total formulation. With this specific ratio of melaleuca alternifolia leaf oil, the stability of topical composition of lanolin has been increased.

According to one embodiment, when polyglyceryl-3- polyricinoleate and melaleuca alternifolia leaf oil have been used together, the composition of lanolin has shown improved stability with their synergistic effect.

In an embodiment of this present invention, the topical composition comprising lanolin, polyglyceryl-3- polyricinoleate and melaleuca alternifolia leaf oil is in the form of diaper rash cream.

In a further embodiment, topical composition of this present invention further comprises Persea gratissima oil (Avocado Oil).

In this embodiment, to further increase the stability and transepidermal penetration capacity of the composition, Persea gratissima oil (Avocado Oil) is used in this present invention. Persea gratissima oil (Avocado Oil) is present in an amount of between 0.01 - 20.0%, preferably 0.01 - 15.0% and more preferably 0.01 - 10.0% by the weight of total formulation. With this specific ratio of Persea gratissima oil (Avocado Oil) stability and transepidermal penetration capacity of the composition are further increased.

Persea Gratissima (Avocado) Oil an oil obtained from the avocado fruit. Because of its chemical composition, avocado oil nourishes deeply, smooths the epidermis and relieves the skin desquamation. Due to the lipids and vitamin A, E and D content of avocado oil, to its extraordinary transepidermal penetration capacity that pass and nourish the different skin layers, to its high moisturizing and emollient power, that smooth and relax the skin. With the synergistic effect of Persea Gratissima (Avocado) Oil with melaleuca alternifolia leaf oil, transepidermal penetration and stability of the topical composition has been improved.

In this embodiment, the ratio of Persea Gratissima oil to melaleuca alternifolia leaf oil which is between 1 - 25 (w/w), preferably 1 - 15 (w/w) and more preferably 1 - 5 (w/w), provides improved transepidermal penetration and stability of topical composition.

In an embodiment of this present invention, the topical composition comprising lanolin, polyglyceryl-3- polyricinoleate, melaleuca alternifolia leaf oil and Persea Gratissima oil is in the form of diaper rash cream.

The topical pharmaceutical composition of this present invention is for use in the treatment of diaper rash.

Suitable emollients are selected from the group consisting of Persea Gratissima oil (avocado oil), cod liver oil, shea butter, lanolin, jojoba oil, argania spinosa kernel oil, aloe vera oil, prunus amydalus dulcis oil, coco-caprylate/caprate, rosmarinus officinalis oil, triticum vulgare extract, helianthus annuus oil, chamomille oil, rice bran oil, foeniculum vulgare oil, jasminum officinale oil, sesame (sesamum indicum) oil, rosehip, centella asiatica extract, cocoa, jojoba butters, cera alba, fumatrice propolis extract, cremerlin pura and mixtures thereof.

Suitable natural humectants are selected from the group consisting of triticum vulgare oil, oenothera biennis oil, daucus carota oil, jojoba oil, calendula officinalis oil, prunus armeniaca oil, vitis vinifera oil, citrus aurantium oil, foeniculum vulgare oil, jasminum officinale oil, tilia cordata flower extract, chamomille oil, sesame (sesamum indicum) oil, aloe vera oil, rosmarinus officinalis oil, red mandarin oil, shea (butyrosperum parkii) butter, lecithin, panthenol (pro-vitamin b5), glycerin and mixtures thereof.

Suitable natural antioxidants are selected from the group consisting of pythrox LT10-IP (lecithin,ascorbyl palmitate+tocopherol rch extract,sunflower oil), vitamin E (tocopheryl asetate/tocopheroll), ascorbic acid (vitamin C) or ascorbyl palmitat propolis extract, allium cepae (onion) bulb extract, glycyrrhiza glabra (organic licorice) root extract and mixtures thereof.

Suitable natural emulsifiers are selected from the group consisting of castile soap, yucca extract, soapwort, quillaja bark extract, lanolin, candelilla, carnauba, jojoba, rice bran, beeswax, lanolin, xanthan gum, quince seed, lesitin, glyceryl oleate, glyceryl stearete citrate and mixtures thereof.

In one embodiment, the topical composition comprising;
a. 1.0 - 80.0% Lanolin,
b. 0.1 - 10.0% Polyglyceryl-3- Polyricinoleate (Akoline PGPR)
c. 0.01 - 5.0% Melaleuca alternifolia leaf oil (Tea Tree oil)
d. 1.0 - 30.0% Emollient,
e. 1.0 - 50.0% Emulsifier and surfactants,
f. 0.001 - 5.0% Antioxidants
g. 0.01 - 5.0% Preservatives by weight of total formulation

### Example 1:

| | **Amount (%)** |
|---|---|
| Lanolin | 12.5 |
| Beeswax (Cera alba) | 1.5 |
| Cremerlin Pura (vegetable oil) | 1.5 |
| Shea Butter (butyrospermum parkii) | 3.0 |
| Prunus amydalus dulcis oil | 3.0 |
| Argania spinosa kernel oil | 3.0 |
| Pythrox LT10-IP (Lecithin+ascorbyl palmitate+tocopherol rch extract+sunflower oil) | 0.20 |
| Polyglyceryl-3- Polyricinoleate (Akoline PGPR) | 5.0 |
| Calendula Officinalis Oil (Calendula Oil CLR) | 1.0 |
| Melaleuca alternifolia leaf oil (Tea Tree oil) | 0.5 |
| Persea gratissima oil (Avocado Oil) | 3.0 |
| Rosmarinus officinalis leaf extract | 0.01 |
| Caprylhydroxamid acid and caprylyl glycol and glycerine (Spektrastat) | 1.0 |
| Centella Asiatica Extract | 1.0 |
| Water | q.s. |

| | |
|---|---|
| q.s.: quantum sufficient | |

The production of the composition is carried out as follows:
**Oil phase:** lanolin, beeswax, cremerlin pura, shea Butter, prunus amydalus dulcis oil, pythrox LT10-IP, argania spinosa kernel oil, polyglyceryl-3- polyricinoleate, calendula officinalis oil, melaleuca alternifolia leaf oil, persea gratissima oil are mixed at 75-80°C.
**Aqueous phase:** Rosmarinus officinalis leaf extract, caprylhydroxamid acid and caprylyl glycol and glycerine and centella asiatica extract are mixed at 75-80°C.

Aqueous phase is added to the oil phase and the mixture is mixed until it cools.

## Claims

1. A topical composition of lanolin comprising polyglyceryl-3- polyricinoleate and one or more excipient.

2. A topical composition of lanolin according to claim 1, wherein lanolin is present in an amount of between 1.0 - 80.0%, preferably 1.0 - 50.0% and more preferably 10.0 - 25.0% by the weight of total formulation.

3. A topical composition of lanolin according to claim 1, wherein polyglyceryl-3-polyricinoleate is present in an amount of between 0.01 - 20.0%, preferably 0.01 - 15.0% and more preferably 0.1 - 10.0% by the weight of total formulation.

4. A topical composition of lanolin according to claim 1 to 3, wherein the ratio of lanolin to polyglyceryl-3- polyricinoleate which is between 1 - 25 (w/w), preferably 1 - 15 (w/w) and more preferably 1 - 5 (w/w) by total weight of formulation.

5. A topical composition of lanolin according to claim 1, said one or more excipient is selected from the group consisting of preservatives, natural emollients, natural humectants, natural antioxidants, natural emulsifiers and natural surfactants.

6. A topical composition of lanolin according to claim 5, wherein said preservatives are selected from the group consisting of melaleuca alternifolia leaf oil, rosmarinus officinalis leaf extract, propylene glycol, phenoxyethanol, methyl paraben, propyl paraben and their salts, sodium benzoate, citric acid, benzoic acid, butylated hydroxytoluene, boric acid, sorbic acid, benzyl alcohol, benzalconium chloride, parahydroxybenzoic acids, butylated hydroxyanisole, hamamelis virginiana extract, tea tree essential oil, thyme essential oil, grapefruit seed extract, bitter orange extract propolis extract, caprylhydroxamid acid, caprylyl glycol, glycerine and mixtures thereof.

7. A topical composition of lanolin according to claim 6, wherein said preservative is melaleuca alternifolia leaf oil.

8. A topical composition of lanolin according to claim 7, wherein melaleuca alternifolia leaf oil is present in an amount of between 0.01 - 12.0%, preferably 0.01 - 7.0% and more preferably 0.01 - 5.0% by the weight of total formulation.

9. A topical composition of lanolin according to claim 1, wherein the topical composition comprising lanolin, polyglyceryl-3-polyricinoleate and melaleuca alternifolia leaf oil is in the form of diaper rash cream.

10. A topical composition of lanolin according to any preceding claims, further comprising persea gratissima oil.

11. A topical composition of lanolin according to any preceding claims, the ratio of persea gratissima oil to melaleuca alternifolia leaf oil which is between 1 - 25 (w/w), preferably 1 - 15 (w/w) and more preferably 1 - 5 (w/w).

12. A topical composition of lanolin according to any preceding claims, for use in the treatment of diaper rash.
